# EUROPÄISCHE PATENTANMELDUNG

(11) **EP 0 779 263 A1**
(43) Veröffentlichungstag der Anmeldung: **18.06.1997**
(21) Anmeldenummer: 96119591.4
(22) Anmeldetag: 06.12.1996
(51) Int. Cl.: C07C 39/27, C07C 43/225, C07C 37/50, C07C 41/18

(54) **Neue Derivate des 2,3,6-Trifluorphenols und ein Verfahren zu ihrer Herstellung**

(30) Priorität: 13.12.1995 DE 19546520
(71) Anmelder: HOECHST AKTIENGESELLSCHAFT, 65929 Frankfurt am Main (DE)
(72) Erfinder: Pfirmann, Ralf, Dr., 64347 Griesheim (DE); Wingen, Rainer, Dr., 65795 Hattersheim (DE)

(57) **Zusammenfassung**

Die vorliegende Erfindung betrifft Verbindungen der Formel (1) 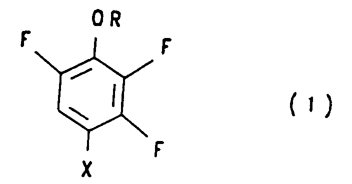 worin R für H, eine geradkettigen oder verzweigten Alkylrest mit 1 bis 6 Kohlenstoffatomen, eine fluorierten geradkettigen oder verzweigten Alkylrest mit 1 bis 6 Kohlenstoffatomen, einen Benzylrest oder einen durch eine Alkylgruppe oder Alkoxygruppe mit jeweils 1 bis 4 Kohlenstoffatom oder Halogen substituierten Benzylrest steht, X für H, Cl, Br oder J steht, und X verschieden von R ist, sowie ein Verfahren zu ihrer Herstellung durch Decarboxylierung einer Verfindung der Formel (2) 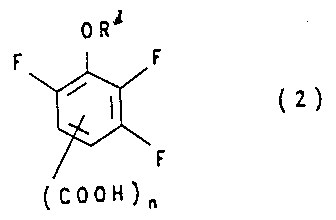 worin R' diesselbe Bedeutung wie R hat, oder, abweichend hiervon, für H steht und n = 1 oder 2 ist, in einem basischen Lösungsmittel oder Lösungsmittelgemisch in Anwesenheit eines Decarboxylierungskatalysators bei 120 bis 220°C decarboxyliert.

## Beschreibung

Die vorliegende Erfindung betrifft neue in 2,3,6-Stellung fluorierte Benzole, die sich vom 2,3,6-Trifluorphenol ableiten. Diese neuen Verbindungen sind für die Herstellung von Flüssigkristallen mit vorteilhaften Eigenschaften von besonderer Bedeutung. Sie lassen sich entsprechend der EP 0 602 596 in die flüssigkristallinen Verbindungen mit vorteilhaften Eigenschaften umwandeln.

Aufgrund der Bedeutung, die flüssigkristallinen Verbindungen, beispielsweise im Rahmen der Datenverarbeitung oder bildlichen Darstellung von Texten oder Zeichnungen zukommt, stellt es eine lohnende Aufgabe dar, neue Verbindungen bereitzustellen, die zur Herstellung von Flüssigkristallen mit vorteilhaften Eigenschaften geeignet sind.

Diese Aufgabe wird gelöst durch Verbindungen der allgemeinen Formel (1) 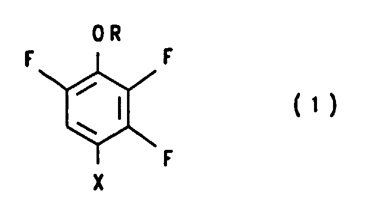 worin R für H, einen geradkettigen oder verzweigten Alkylrest mit 1 bis 6 Kohlenstoffatomen, einen fluorierten geradkettigen oder verzweigten Alkylrest mit 1 bis 6 Kohlenstoffatomen, einen Benzylrest oder einen durch eine Alkylgruppe oder Alkoxygruppe mit jeweils 1 bis 4 Kohlenstoffatomen oder durch Halogen substituierten Benzylrest steht, X für H, Cl, Br oder I steht und X verschieden von R ist.

Die neuen Verbindungen weisen aufgrund ihrer Substitution in der 1,2,3,6-Stellung bzw. 1,2,3,4,6-Stellung ein ungewöhnliches Substitutionsmuster auf.

In den Verbindungen der allgemeinen Formel (1) steht R insbesondere für H, einen geradkettigen oder verzweigten Alkylrest mit 1 bis 6 Kohlenstoffatomen oder einen Benzylrest, bevorzugt für einen geradkettigen oder verzweigten Alkylrest mit 1 bis 4 Kohlenstoffatomen oder einen Benzylrest, besonders bevorzugt für einen Alkylrest mit 1 bis 2 Kohlenstoffatomen oder einen Benzylrest.

In den Verbindungen der allgemeinen Formel (I) steht X insbesondere für H, Cl oder Br, bevorzugt für H und Br, besonders bevorzugt Br.

Von besonderem Interesse sind die Verbindungen der Formel (1) 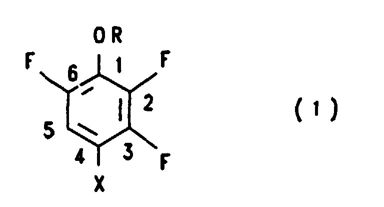 worin R für einen Methyl- oder Benzylrest steht und X für H, Cl, Br oder J, insbesondere für H, Cl oder Br steht. Hierbei handelt es sich um das 2,3,6-Trifluoranisol und das 2,3,6-Trifluorbenzyloxybenzol und deren in 4-Stellung halogenierten Derivate. Von besonderem Interesse sind auch Verbindungen der Formel (1) worin R für H und X für Cl, Br oder J steht, insbesondere R für H und X für Br steht. Hierzu zählen 4-Chlor-2,3,6-trifluorphenol, 4-Brom-2,3,6-trifluorphenol und 2,3,6-Trifluor-4-jodphenol.

Da für die Herstellung der neuen Derivate des 2,3,6-Trifluorphenols kein Verfahren existiert, besteht ein dringender Bedarf, ein geeignetes Verfahren zu ihrer Herstellung bereitzustellen, das die gewünschten Produkte auf einfache Art und ohne großen technischen Aufwand in hoher Ausbeute zugänglich macht.

Diese Aufgabe wird gelöst durch ein Verfahren zur Herstellung der Verbindungen der Formel (1), worin R und X die vorstehend genannte Bedeutung besitzen. Es ist dadurch gekennzeichnet, daß man eine Verbindung der allgemeinen Formel (2) 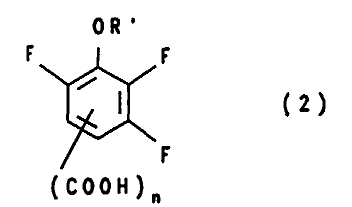 worin R' diesselbe Bedeutung wie R hat, oder abweichend hiervon, für H steht und n = 1 oder 2, ist, in einem basischen Lösungsmittel oder Lösungsmittelgemisch in Anwesenheit eines Decarboxylierungskatalysators bei 120 bis 220°C decarboxyliert, das Reaktionsgemisch gegebenenfalls ansäuert, das decarboxylierte Produkt abtrennt und gegebenenfalls in das decarboxylierte Produkt den Rest X = Cl, Br oder J durch Halogenierung und gegebenfalls den Rest R, sofern dieser nicht H ist, durch Veretherung einführt.

In einer Vielzahl von Fällen hat es sich bewährt, eine Verbindung der Formel (2), worin R' diesselbe Bedeutung wie R hat, einzusetzen.

Die Decarboxylierung mehrfach halogenierter, respektive mehrfach fluorierter Benzoesäuren verläuft in nicht vorhersehbarer Weise höchst unterschiedlich und liefert die entsprechenden decarboxylierten Produkte in sehr unterschiedlichen Ausbeuten. Während die Decarboxylierung von 2,3,4,5-Tetrafluorbenzoesäure das 1,2,3,4-Tetrafluorbenzol in 60 bis 62 % Ausbeute ergibt, führt die Decarboxylierung von 2-Chlor-4,5-difluorbenzoesäure lediglich zu einer Ausbeute von 1 % an 1-Chlor-3,4-difluorbenzol. (Siehe auch N.J. O'Reilly, ACS 10^{th} Winter Fluorine Symposium, St. Petersburg, Florida 1991).

Vor diesem Hintergrund ist es als überraschend anzusehen, daß die Decarboxylierung der Verbindungen der Formel (2) zu entsprechenden decarboxylierten Produkten in Ausbeuten von 65 bis 90 % führt. Hierbei ist insbesondere zu berücksichtigen, daß die Carbonsäuren der Formel (2) hinsichtlich der Fluorsubstituenten ein Substitutionsschema aufweisen, das dem Substitutionsschema der Halogene in der 2-Chlor-4,5-difluorbenzoesäure recht ähnlich ist. In beiden Fällen sind die drei Fluor- bzw. drei Halogenreste unsymmetrisch am Benzolkern angeordnet, wie beispielsweise aus den nachfolgenden Formeln (A), (B) und (C) hervorgeht.

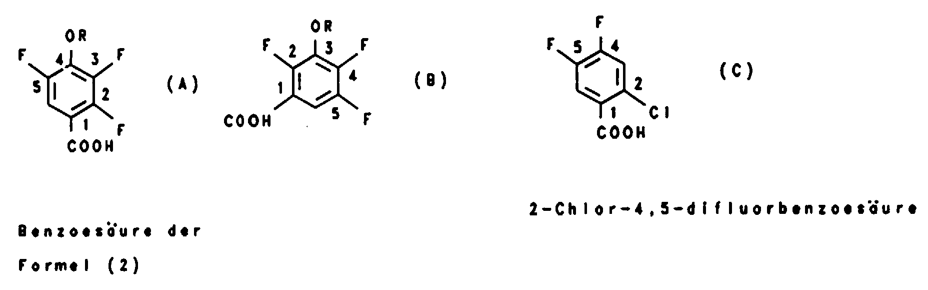

In der Verbindung der Formel (A) sind die Fluorsubstituenten in 2,3,5-Position angeordnet, während in der Verbindung der Formel (B) die Fluoratome diesselbe Anordnung wie die Halogene in der 2-Chlor-4,5-difluorbenzoesäure aufweisen und dementsprechend die 2,4,5-Position besetzen.

Die für die Herstellung der erfindungsgemäßen Verbindungen der Formel (1) benötigten Ausgangsstoffe der Formel (2) lassen sich, wie in EP 602 549 oder EP 271 275 beschrieben, herstellen.

Das erfindungsgemäße Verfahren läßt sich mit gutem Erfolg durchführen, wenn man eine Verbindung der Formel (2), worin n = 1 ist, einsetzt.

Im allgemeinen setzt man ein basisches und dipolar aprotisches Lösungsmittel, ein Alkylamin mit 6 bis 30 Kohlenstoffatomen, ein Dialkylamin mit 6 bis 30 Kohlenstoffatomen je Alkylrest, ein Trialkylamin mit 4 bis 30 Kohlenstoffatomen je Alkylrest, eine N-enthaltende heterocyclische Verbindung oder ein Gemisch dieser vorstehend genannten Stoffe als basisches Lösungsmittel oder Lösungsmittelgemisch ein.

Als basisches dipolar aprotisches Lösungsmittel eignen sich N-Methylpyrrolidon, Dimethylacetamid und 1,3-Dimethylimidazolidin-2-on oder Mischungen dieser Stoffe.

In einer Reihe von Fällen hat es sich bewährt, ein Alkylamin mit 8 bis 20 Kohlenstoffatomen, ein Dialkylamin mit 8 bis 20 Kohlenstoffatomen je Alkylrest, ein Trialkylamin mit 6 bis 20 Kohlenstoffatomen je Alkylrest, insbesondere ein Alkylamin mit 8 bis 14 Kohlenstoffatomen, ein Dialkylamin mit 8 bis 16 Kohlenstoffatomen je Alkylrest, ein Trialkylamin mit 6 bis 14 Kohlenstoffatomen je Alkylrest oder Mischungen dieser Amine einzusetzen. Als N-enthaltende heterocyclische Verbindung verwendet man Pyridin, ein alkyliertes Pyridin, Chinolin, ein alkyliertes Chinolin, Isochinolin, ein alkyliertes Isochinolin oder Gemische dieser Stoffe.

Eine Verfahrensvariante besteht darin, ein basisches Lösungsmittel und Wasser als Lösungsmittelgemisch einzusetzen.

Als Decarboxylierungskatalysator setzt man Kupfer, eine Kupfer(I)verbindung, eine Kupfer(II)verbindung, beispielsweise Kupfer(I)oxid, Kupfer(II)oxid, Kupfer(I)sulfat, Kupfer(II)sulfat, Kupfer(I)chlorid, Kupfer(II)chlorid, Kupfer(I)fluorid, Kupfer(II)fluorid, Kupfercarbonat, Kupfer(I)hydroxid oder Kupfer(II)hydroxid ein.

Der Decarboxylierungskatalysator gelangt in eine Menge von 0,1 bis 10, insbesondere 0,3 bis 3 Gew.-% bezogen auf die Verbindung der Formel (2) zum Einsatz.

In einer Reihe von Fällen ist es vorteilhaft, die Decarboxylierung bei einem pH-Wert von 5 bis 8 durchzuführen. Dies gilt für den Fall, daß sich der pH-Wert messen läßt, beispielsweise bei Verwendung wasserhaltiger Solventien.

Die Decarboxylierung läßt sich mit gutem Erfolg bei einer Temperatur von 130 bis 190, insbesondere 140 bis 170°C durchführen.

Nach Beendigung der Decarboxylierung säuert man das Reaktionsgemisch, beispielsweise durch Zugabe einer Mineralsäure wie Salzsäure, Schwefelsäure oder Phosphorsäure an und trennt das decarboxylierte Produkt mittels Extraktion oder Wasserdampfdestillation ab. In einigen Fällen hat sich die Wasserdampfdestillation als besonders schonende Methode zur Abtrennung bewährt.

Das Ansäuern ist erforderlich, falls die im Reaktionsgemisch enthaltende Verbindung der Formel (1) saure Gruppen besitzt.
Das Ansäuern des Reaktionsgemisches kann entfallen, falls die Verbindung der Formel (1) keine sauren Gruppen enthält, beispielsweise falls R nicht für H steht.

Beabsichtigt man, das decarboxylierte Produkt mittels Extraktion abzutrennen, so verwendet man ein wasserunlösliches organisches Lösungsmittel oder Lösungsmittelgemisch. Geeignet als Lösungsmittel sind chlorierte aliphatische Kohlenwasserstoffe, beispielsweise Dichlormethan, Chloroform, 1,2-Dichlorethan, Trichlorethylen, Perchlorethylen, aromatische Kohlenwasserstoffe, beispielsweise Toluol, o-Xylol, m-Xylol, p-Xylol, Gemische isomerer Xylole, Mesitylen, Chlorbenzol, Dichlorbenzol, isomere Dichlorbenzole, Dichlortoluol, Chlortoluol oder Gemische der vorstehend genannten Solventien. Geeignet sind auch aliphatische Kohlenwasserstoffe beispielsweise n-Pentan, n-Hexan, n-Heptan, deren Isomere, Benzine, Petrolether oder Gemische dieser Solventien.

Soll das decarboxylierte Produkt, beispielsweise niedere Ether des 2,3,6-Trifluorphenols oder das als Vorprodukt zur nachfolgenden Halogenierung oder Veretherung benötigte 2,3,6-Trifluorphenol, durch Wasserdampfdestillation abgetrennt werden, so kann man dem decarboxylierten Produkt Wasser zusetzen und das dabei anfallende Wasser enthaltende Gemisch destillieren oder direkt Wasserdampf einleiten und das gewünschte Produkt mit dem eingeleiteten Wasserdampf überdestillieren.
Die Wasserdampfdestillation hat den Vorteil, daß das decarboxylierte Produkt bereits in vorgereinigter Form anfällt und einer weiteren Reinigung zumeist nicht mehr bedarf.

Der Rest X = Cl, Br oder J wird durch Halogenierung des decarboxylierten Produktes eingeführt. Die Halogenierung kann in Substanz, das heißt in Abwesenheit eines Lösungsmittels, oder in Anwesenheit eines Lösungsmittels durchgeführt werden. Geeignete Lösungsmittel sind organische Lösungsmittel, die unter den Bedingungen der Halogenierung inert sind, und Wasser. Geeignete organische Lösungsmittel sind aliphatische Kohlenwasserstoffe, beispielsweise Hexan, Heptan, Octan, halogenierte aliphatische Kohlenwasserstoffe, beispielsweise Dichlormethan, Chloroform, Dichlorethan, Perchlorethylen, oder halogenierte aromatische Kohlenwasserstoffe, beispielsweise Chlorbenzol, Dichlorbenzol, Gemische isomerer Dichlorbenzole, Trichlorbenzol, Gemische isomerer Trichlorbenzole sowie Chlortoluol, Dichlortoluol, Trichlortoluol und deren isomere Gemische. Auch Mischungen der vorstehend genannten Lösungsmittel lassen sich verwenden.

Die Halogenierung läßt sich in Anwesenheit eines Halogenierungskatalysators durchführen. Geeignet als Halogenierungskatalysator sind beispielsweise Eisen, Eisen(II), Eisen(III)halogenide, Jod oder Jodhalogenide und deren Gemische. Obwohl zu erwarten wäre, daß der aromatische Ring im decarboxylierten Produkt wegen der drei am aromatischen Ring befindlichen Fluoratome im erheblichen Maße desaktiviert sein sollte, erweist sich das decarboxylierte Produkt in überraschender Weise reaktiv. Es ist nämlich möglich, die Halogenierung auch in Abwesenheit eines Halogenierungskatalysators durchzuführen. Üblicherweise wird man dieser Variante der Halogenierung den Vorzug geben.

Als Halogenierungsmittel kann man das Halogen in elementarer Form oder als Hypohalogenit einsetzen. Besonders einfach gestaltet sich die Halogenierung unter Verwendung von Hypohalogenit, beispielsweise in Form einer Halogenbleichlauge, insbesondere Chlorbleichlauge oder Brombleichlauge. Man setzt das Halogen in einer Menge von 80 bis 200, insbesondere 100 bis 120 % der stöchiometrischen erforderlichen Menge ein und führt die Halogenierung bei einer Temperatur von -10 bis 80, insbesondere 0 bis 50, bevorzugt 10 bis 40°C durch.
Die Konzentration des in organischen Lösungsmitteln und/oder Wasser als Ausgangsmaterial verwendeten decarboxylierten Produktes kann in weiten Bereichen variiert werden.

Arbeitet man im wäßrigen Medium, so ist es besonders bei Verwendung von 2,3,6-Trifluorphenol, aber auch von anderen decarboxylierten Produkten von Vorteil, in Anwesenheit von 1 bis 10, insbesondere 2 bis 5 Moläquivalenten Hydroxid, bezogen auf 2,3,6-Trifluorphenol oder decarboxyliertes Produkt, zu arbeiten. Als Hydroxidquelle kommen Lösungen von Alkali- oder Erdalkalimetallhydroxiden oder basisch wirkende Substanzen in Betracht.

Den Rest R kann man, sofern R nicht H ist, durch Veretherung einführen. Hierzu kann man das decarboxylierte Produkt in Form des Phenolats mit einer Halogenverbindung R-Hal im Sinne einer Williamson'schen Ethersynthese oder mit einem Sulfat der Formel R-O-SO₂-O-R umsetzen. Siehe auch T. Greene, Protective Groups in Organic Chemistry (1991) (John Wiley & Sons) Abschnitt aromatische Ether, Seiten 143 bis 174.

Bei Durchführung des erfindungsgemäßen Verfahrens ist man hinsichtlich Halogenierung und Veretherung des decarboxylierten Produktes an keine Reihenfolge gebunden. Man kann nach Belieben nur die Halogenierung oder nur die Veretherung durchführen. Man kann aber auch zunächst die Halogenierung und anschließend die Veretherung oder umgekehrt erst die Veretherung und anschließend die Halogenierung durchführen.
Es ist möglich, auf die Veretherung und gegebenenfalls die Halogenierung zu verzichten, wenn man eine Verbindung der Formel (2), worin R' dieselbe Bedeutung wie R in Formel (1) hat, aber nicht für H steht, in das erfindungsgemäße Verfahren einsetzt und lediglich decarboxyliert. In diesen Fällen besitzt das decarboxylierte Produkt bereits einen Ethersubstituenten. Falls gewünscht, kann dieses Produkt anschließend noch halogeniert werden.

Das Verfahren läßt sich kontinuierlich oder diskontinuierlich bei Atmosphärendruck, Unterdruck oder Überdruck durchführen.

Die nachfolgenden Beispiele beschreiben die Erfindung näher, ohne sie darauf zu beschränken.

### Experimenteller Teil:

### Beispiel 1a

### Herstellung von 2,3,6-Trifluorphenol (Vorprodukt)

Man löst 74,2 g (0,386 mol) farblose bis hellbeige 4-Hydroxy-2,3,5-trifluorbenzoesäure (hergestellt gemäß EP 602 549) in 350 g N-Methylpyrrolidon, setzt 1 g Kupfer(I)oxid zu und erhitzt die Mischung unter Rühren 3 Stunden auf 155°C. Nach Beendigung der gleichmäßig verlaufenden Gasentwicklung wird noch 1 Stunde bis zu einer Temperatur von 170°C nachgerührt.

Nach Abkühlen gibt man zu der Mischung 500 g Wasser und 200 g 96 %ige Schwefelsäure und destilliert das decarboxylierte Produkt mit Wasser ab. Aufgrund der hohen Wasserlöslichkeit des 2,3,6-Trifluorphenols bildet sich eine organische Phase nur im geringen Umfange. Das Destillat wird mit jeweils 100 g Dichlormethan extrahiert, die vom Wertprodukt befreite Wasserphase wird in die Destillation zurückgeführt. Dieser Vorgang wird solange (6 bis 8 mal) wiederholt, bis im Sumpf kein wesentlicher Produktanteil nachgewiesen werden kann.
Die vereinigten organischen Phasen werden über Magnesiumsulfat getrocknet, filtriert und das Dichlormethan wird am Rotationsverdampfer entfernt. Man erhält 56 g eines gelb-orangen Öls, entsprechend 73 % Ausbeute, das noch ca. 25 % N-Methylpyrrolidon (GC-Analyse) enthält.
Das Rohprodukt wird zusammen mit dem aus Beispiel 1 b erhaltenen Rohprodukt aufgearbeitet.

### Beispiel 1 b

### Herstellung von 2,3,6-Trifluorphenol (Vorprodukt)

Man löst 144,1 g (0,75 mol) 4-Hydroxy-2,3,5-trifluorbenzoesäure (hergestellt gemäß EP 602 549) in 500 g N-Methylpyrrolidon, setzt 2 g Kupfer(I)oxid zu und erhitzt die Mischung unter Rühren auf 155°C. Nach 3 Stunden ist laut HPLC-Analyse noch kein vollständiger Umsatz erzielt. Man setzt 250 g N-Methylpyrrolidon, 4 g Kupfer(I)oxid und 2,5 g Wasser zu und erhitzt unter intensivem Rühren weitere 3,5 Stunden auf 180°C. Im Reaktionsgemisch läßt sich danach keine 4-Hydroxy-2,3,5-trifluorbenzoesäure nachweisen.
Man kühlt die dunkelrote Suspension ab und gießt sie auf ein Gemisch aus 1000 g Wasser und 185 g 30 Gew.-%ige Salzsäure (entsprechend 1,5 Mol). Die Mischung wird 5 mal mit je 100 g Dichlormethan extrahiert, die vereinten organischen Phasen einschließlich des aus Beispiel 1a erhaltenen Rohproduktes werden 2 mal mit 50 g Wasser gewaschen, über Magnesiumsulfat getrocknet und anschließend wird das Dichlormethan im Rotationsverdampfer entfernt. Man erhält 126 g rohes gelboranges 2,3,6-Trifluorphenol, das gemäß GC-Analyse bereits in 95 %iger Reinheit vorliegt.
Dieses Rohprodukt wird unter Zusatz von Diphenylsulfon als Sumpf über eine 25 cm lange Vigreux-Kolonne fraktioniert destilliert. Man erhält bei 8 Torr (11,4 mbar) und 98 bis 110°C Kopftemperatur 114,5 g (0,774 Mol) 2,3,6-Trifluorphenol, entsprechend einer auf die beiden Beispiele 1 a und 1b bezogenen Ausbeute von 68 %, als farbloses Öl, das zu einem äußerst flüchtigen, bei etwa 40°C schmelzenden, farblosen Feststoff mit starker Sublimationsneigung erstarrt. Die Sumpftemperatur durchläuft während der Destillation ein Temperaturintervall von 120 bis 160°C.

MS: m/z (%)* = 44, 49, 50 (17,3), 51 (5,3), 53, 55, 56 (5,9), 57 (4,8), 60, 61 (3,8), 62, 63, 64, 68 (4,3), 69 (10,2), 70 (2,4), 71 (2,7), 73, 74 (5,2), 75 (8,9), 77, 79 (2,7), 80 (3,9), 81 (12,7), 82, 86, 87, 88, 91, 93 (3,6), 94, 98, 99 (39,8), 100 (73,4), 101 (7,7), 117, 119 (15,0), 120, 128 (13,1), 129, 147 (2,9), 148 (100), 149 (6,9).
∗ Peaks ohne Mengenangabe liegen im Bereich von jeweils 0,6 bis 1,9 %

### 2,3,6-Trifluorphenol

### ¹H-NMR (CDCl₃, TMS)

- δ [ppm] =: 5,24 (s (br), 1H-OH)
6,69 (cm, 1H, Ar-H⁴)
6,84 (cm, 1H, Ar-H⁵)

### ¹⁹F-NMR (CDCl₃, CFCl₃)

- δ [ppm ]: = -141,25 (m, 1F, ArF⁶)
-141,74 (dddd, 1F, J = 7,6 Hz; 9,2 Hz, 13,7 Hz, 19,8 Hz, Ar-F³)
-157,74 (dddd, 1F, J = 7,6 H, 19,9 Hz, Ar-F²)

### Beispiel 2

### Herstellung von 2,3,6-Trifluoranisol

Man legt 100 g (0,485 Mol) 3-Methoxy-2,4,5-trifluorbenzoesäure (hergestellt durch Alkylierung von 3-Hydroxy-2,4,5-trifluorbenzoesäure), 60 g N-Methylpyrrolidon und 0,5 g Kupfer(I)oxid vor und erhitzt die rote Mischung unter Rühren 1,5 Stunden auf 160 bis 166°C. Die Beendigung der Gasentwicklung zeigt ebenso wie die HPLC-Analyse einen vollständigen Umsatz an.
Anschließend destilliert man bei Normaldruck und erhält etwa 50 g einer farblosen Flüssigkeit. Der Sumpf wird verworfen.

Die wäßrige Phase des Destillats (Restwasser aus N-Methylpyrrolidon) wird mit 10 g Dichlormethan extrahiert und mit der organischen Phase vereint und anschließend wird die gesamte organische Phase mit 50 ml Wasser gewaschen, um N-Methylpyrrolidon zu entfernen. Man trocknet und filtriert die organische Phase und entfernt noch vorhandene Lösungsmittelanteile.
Man erhält 58 g (0,358 Mol) farbloses 2,3,6-Trifluoranisol in einer Reinheit von 95 %, bestimmt durch GC-Analyse. Dies entspricht einer Ausbeute von 70 %.

MS: m/z (%)* = 49, 50 (3,5), 51 (4,2), 55, 56 (2,7), 57 (3,9), 61 (3,3), 62 (2,8), 63 (3,0), 68 (3,9), 69 (12,2), 71, 74 (2,5), 75 (9,3), 79, 80 (3,2), 81 (17,7), 82, 83, 84 (2,6), 86, 87, 88, 92, 93 (7,2), 95, 98, 99 (9,8), 100 (3,5), 101, 111, 112, 113 (4,6), 114 (3,3), 115, 117, 119 (100), 120 (6,0), 128, 130, 131 (6,8), 132 (2,6), 133 (3,5), 147 (61,5), 148 (4,6), 159, 162 (99,5), 163 (8,3).
* Peaks ohne Mengenangabe < 2 %

### 2,3,6-Trifluoranisol:

### ¹H-NMR (CDCl₃, TMS):

- δ [ppm] =: 4,04 (ct, 3H, Ar-OCH₃)
6,74 - 6,88 (m, 2H, Ar-H^{4,5})

### ¹⁹F-NMR (CDCl₃, CFCl₃):

- δ [ppm] =: -134,25 (cm, 1F, Ar-F⁶)
-141,6 (dddd, 1F, Ar-F³)
-152,15 (dcm, 1F, Ar-F²)

### Beispiel 3

### Herstellung von 4-Chlor-2,3,6-trifluorphenol

Man löst 10 g (0,0678 Mol) 2,3,6-Trifluorphenol (hergestellt gemäß Beispiel 1a und 1 b) in 50 ml Dichlormethan und versetzt diese Lösung bei 20°C mit 20 g einer 30 %igen wäßrigen Natronlauge. Anschließend gibt man 60 g einer 14,8 %igen Chlorbleichlauge (0,119 mol, durch Titration bestimmt), die aus 100 g NaOH, 240 g Wasser und 57 g Chlor hergestellt wird, zu und rührt bei 20°C über einen Zeitraum von 48 Stunden kräftig.
Anschließend wird überschüssige Chlorbleichlauge durch Zusatz von Natriumdithionit zerstört, das Reaktionsgemisch bis pH = 2 angesäuert, die das 4-Chlor-2,3,6-trifluorphenol enthaltende organische Phase abgetrennt und über MgSO₄ getrocknet.
Nach Filtration und Entfernen des Lösungsmittels erhält man 10,2 g Rohprodukt, das 4-Chlor-2,3,6-trifluorphenol in einer Reinheit > 93 %, bestimmt durch GC-Analyse, entsprechend 55,9 mMol, enthält.
Dies entspricht einer Ausbeute von 82,5 %, bezogen auf eingesetztes 4-Chlor-2,3,6-trifluorphenol.

### Beispiel 4

### Herstellung von 4-Brom-2,3,6-trifluorphenol

Man löst 10 g (0,0678 Mol) 2,3,6-Trifluorphenol (hergestellt gemäß Beispiel 1a und 1b) in 50 ml Dichlormethan und versetzt diese Lösung bei 20°C mit 13 g (0,0811 mol) Brom. Anschließend vermischt man durch Umschütteln und läßt das Gemisch 7 Tage stehen.
Der Umsatz, bezogen auf eingesetztes 2,3,6-Trifluorphenol, beträgt gemäß GC-Analyse über 95 %, wobei zu etwa 10 % eine Dibromierung stattgefunden hat.
Das Gemisch wird durch Zugabe von 5 %iger wäßriger Natriumhydrogencarbonat-Lösung entsäuert. Man trennt die organische Phase ab und trocknet sie über MgSO₄. Das dabei anfallende Gemisch wird in einem Rotationsverdampfer vom Lösungsmittel befreit und der verbleibende Rückstand (16,6 g] wird über eine 30 cm lange Vigreux-Kolonne fraktioniert destilliert.

Man erhält bei 5 Torr (6,7 mbar) und einer Kopftemperatur von 68 bis 75°C 10,8 g (0,0472 mol) 4-Brom-2,3,6-trifluorphenol in einer Reinheit von 95 %, bestimmt durch GC-Analyse. Dies entspricht eine Ausbeute von 70,2 %, bezogen auf eingesetztes 2,3,6-Trifluorphenol. Die Sumpftemperatur durchläuft während der Destillation eine Temperatur von 105 bis 190°C.

### 4-Brom-2,3,6-trifluorphenol

### ¹H-NMR (CDCl₃, TMS):

- δ [ppm] =: 5,4 (br), 1H, Ar-OH)
7,13 (ddd, 1H, J_{AD} = 2,8 Hz, J_{AB} = 6 Hz, J_{AC} = 9,7 Hz, Ar-H⁵ (A))

### ¹⁹F-NMR (CDCl₃, CFCl₃):

- δ [ppm] =: -133,65 (ddd, 1F, J_{AB} = 6 Hz, J_{BC} = 10,4 Hz, J_{BD} = 21,3 Hz, Ar-F³ (B))
-139,76 (ddd, 1F, J_{CD} = 6,9 Hz, J_{AC} = 9,7 Hz, J_{BC} = 10,4 Hz, Ar-F⁵ (C))
-154,31 (ddd, 1F, J_{AD} = 2,8 Hz, J_{CD} = 6,9 Hz, J_{BD} = 21,3 Hz, Ar-F⁴ (D))
- IR : v [cm⁻¹] =: 720, 825, 850, 985, 1080, 1190, 1230, 1320, 1350, 1480, 1510, 1615, 3080, 3430 (br), 3570
- MS: m/z (%) =: 49, 50, 51 (2,7), 55, 56 (3,3), 61 (2,8), 68 (6,1), 69 (10,2), 71 (2,6), 74, 75 (6,7), 79 (7,3), 80 (7,3), 81 (3,3), 87, 93 (3,5), 98 (4,5), 99 (53,1), 100 (3,5), 113, 114, 117 (2,7), 118 (5,3), 119 (15,5), 130, 146, 147 (3,5), 178 (20,6), 179 (2,0), 180 (20,8), 181, 197 (4,8), 199 (5,2), 206 (4,0), 208 (5,3), 226 (92,7), 227 (8,4), 228 (100), 229 (6,4)

### Beispiel 5

### Herstellung von 2,3,6-Trifluorisopropoxybenzol

Man legt bei 20 bis 25°C 14,3 g (0,1 Mol) 2,3,6-Trifluorphenol (hergestellt gemäß Beispiel 1a und 1b) und 200 ml Dimethylformamid vor und fügt unter Rühren bei dieser Temperatur 27,5 g (0,194 Mol) gekörntes Kaliumcarbonat (99 %ig) und 42,3 g (0,344 Mol) 2-Brompropan (99 %ig) zu. Es entsteht eine farblose Suspension, die nach 3 Stunden 2,3,6-Trifluorisopropoxybenzol entsprechend einem Umsatz von 94 % (bestimmt durch GC-Analyse) enthält und eine rosa Färbung aufweist. Man rührt über Nacht (20 Stunden) nach und gibt die gesamte Mischung zu 400 g Wasser. Unter leichter Wärmetönung setzt sich eine untere, rosagefärbte organische Phase, die das Wertprodukt enthält, ab. Die Phasen werden getrennt und der Wasserphase wird zweimal mit je 30 ml Dichlormethan extrahiert. Die Dichlormethan-Phase und die organische Phase werden vereint, über Na₂SO₄ getrocknet und filtriert. Anschließend entfernt man die Lösungsmittel im Rotationsverdampfer.
Mittels fraktionierter Destillation erhält man drei Fraktionen (insgesamt 13,5 g, entsprechend 71 mMol) 2,3,6-Trifluorisopropoxybenzol (mittlerer Reingehalt 94,2 %, bestimmt durch GC-Analyse).

### 2,3,6-Trifluorisopropoxybenzol

- MS: m/z (%) =: 27 (7,0), 31, 38, 39 (7,4), 40, 41 (11,1), 42 (2,1), 43 (9,2), 50 (2,4), 51 (2,6), 57, 69 (4,5), 75 (3,6), 81 (5,4), 93, 99 (5,5), 100, (13,9), 101, 119 (14,0), 127, 128 (4,4), 131 (2,2), 148 (100), 149 (7,3), 175 (6,9), 190 (M⁺ ; 4,7)

### ¹H-NMR (CDCl₃, TMS)

- δ [ppm] =: 1,36 (d, 6H, -OCH(CH₃)₂)
4,49 (tr/hpt, 1H, ArOCH-)
6,82 (m, 2H, Ar-H^{4,5})

### ¹⁹F-NMR (CDCl₃, CFCl₃)

- δ [ppm] =: -132,80 (m, 1F, J = 4,6 Hz; 13,0 Hz, Ar-F⁶)
-141,80 (dddd, 1F, J = 13,0 Hz; 20,4 Hz, Ar-F³)
-150,40 (dm, 1F, J = 4,6 Hz, 20,4 Hz, Ar-F²)

### Beispiel 6

### Herstellung von 1,3,4-Trifluor-2-(2'-fluorbenzyloxy)-benzol

Man legt unter Argonatmosphäre 200 ml N,N-Dimethylformamid, 27,5 g (0,194 Mol) Kaliumcarbonat, 14,3 g (0,1 Mol) 96,8 %iges 2,3,6-Trifluorphenol (hergestellt gemäß Beispiel 1a und 1b) und 49,7 g (0,344 Mol) 2-Fluorbenzylchlorid unter Rühren bei 20 bis 25°C vor. Die ursprüngliche farblose Suspension verfärbt sich. Nach zwei Stunden enthält die inzwischen beigebraune Suspension kein 2,3,6-Trifluorphenol mehr (bestimmt durch GC-Analyse).
Nach 5,5 Stunden gibt man die gesamte Mischung zu 400 g Wasser, wobei sich eine trübe, braune organische Phase absetzt. Man trennt die organische und die wäßrige Phase und extrahiert die wäßrige Phase zweimal mit je 40 ml Dichlormethan. Die dabei anfallende organische Phase enthält 53 % Dimethyformamid, 35 % 2-Fluorbenzylchlorid und lediglich 13 % 1,3,4-Trifluor-2-(2'-fluorbenzyloxy)-benzol. Wegen der geringen Menge Wertprodukte wird auf eine Weiterverarbeitung dieser organischen Phase verzichtet.
Die zuvor bei der Zugabe zu Wasser abgeschiedene organische Phase wird zweimal mit je 30 g Wasser gewaschen, von der wäßrigen Phase abgetrennt und über Na₂SO₄ getrocknet.

Durch fraktionierte Destillation erhält man als Vorlauf bei 1 bis 2 Torr (1,3 bis 2,6 mbar) und 39°C Kopftemperatur 24,3 g 2-Fluorbenzylchlorid zurück. Anschließend fallen 7,7 g (40 % 2-Fluorbenzylchlorid, 60 % 1,3,4-Trifluor-2-(2'-fluorbenzyloxy)-benzol) als Mischfraktion an, die wie das reine Produkt (99 %, bestimmt durch GC-Analyse) bei 1 bis 2 Torr (1,3 bis 2,6 mbar) und einer Kopftemperatur von 104°C übergeht.
Man erhält 20,1 g (78,5 mMol entsprechend 78,5 % Ausbeute) 1,3,4-Trifluor-2-(2'-fluorbenzyloxy)-benzol als farbloses Öl. Unter Einbeziehung der als Zwischenlauf angefallenen Mischfraktion beträgt die Ausbeute 24,7 g (96,5 mMol entsprechend 96,5 % Ausbeute).
Es verbleibt 1 g Sumpf.
- MS: m/z (%) =: 28, 31 (2,2), 38, 39 (4,7), 43, 50, (2,8), 51 (3,9), 56, 57 (7,5), 58, 59 (2,1), 61, 62 (2,3), 63 (5,4), 68, 69 (4,1), 70, 74, 75 (3,7), 80, 81 (4,6), 83 (21,5), 84, 88, 89 2,8), 93, 95, 99, 100, 107 (4,6), 109 (100), 110 (8,6), 119 (9,9), 120, 123, 131, 147 (1,7), 206 (0,2), 256 (M⁺, 1,9), 257

### ¹H-NMR (CDCl₃, TMS)

- δ [ppm] =: 5,28 (s, 2H, Ar-O-CH₂-Ar)
6,78 - 6,86 (m, 2H)
7,06 (tm, 1H)
7,15 (tm, 1H)
7,28 - 7,38 (m, 1H)
7,50 (tm, 1H)

### ¹⁹F-NMR (CDCl₃, CFCl₃)

- δ [ppm] =: -118,90 (m, 1F, J = 1,5 Hz; 1,5 Hz, Ar-F^{2'})
-132,80 (m, 1F, J = 3,8 Hz; 1,5 Hz; 13,0 Hz, Ar-F⁶)
-141,40 (cm, 1F, J = 13,0 Hz; 20,2 Hz, Ar-F³)
-150,50 (m, 1F, J = 1,5 Hz; 3,8 Hz, 20,2 Hz, Ar-F²)

### Beispiel 7

### Herstellung von 2,3,6-Trifluorphenol (Vorprodukt) ausgehend von 4-Hydroxy-3,5,6-trifluorphthalsäure

### a) Herstellung von 3-Hydroxy-2,4,5-trifluorbenzoesäure (Vorprodukt)

450,3 g einer stark alkalischen, wäßrigen Lösung, die 41,8 g (0,177 Mol) 4-Hydroxy-3,5,6-trifluorphthalsäure in Form entsprechender Alkalisalze enthält, und 0,4 g Kupfer(I)oxid, werden mit 12,6 g eines Gemisches verschiedener aliphatischer Trialkylamine mit jeweils 6 bis 14 Kohlenstoffatomen im Alkylrest (Hostarex A 327; ein Handelsprodukt der Hoechst AG) versetzt und mit insgesamt 166,8 g einer 30 %igen wäßrigen Salzsäure auf pH 5 eingestellt und anschließend über einen Zeitraum von 6 Stunden bei 105°C unter Rühren erwärmt. Der pH-Wert verändert sich infolge der Decarboxylierung und wird nach einer Stunde durch Zugabe von 23,4 g 30 %iger wäßriger Salzsäure und nach 3 Stunden durch Zugabe von 15,9 g 30 %iger wäßriger Salzsäure entsprechend korrigiert (auf pH 5 eingestellt).
Man kühlt ab, stellt einen pH-Wert von 8 ein und trennt die wäßrige Phase (601 g), die 31,3 g (91,3 % der Theorie) 3-Hydroxy-2,4,5-trifluorbenzoesäure (bestimmt durch kalibrierte HPLC-Chromatographie) enthält, ab. Anschließend stellt man die wäßrige Phase auf einen pH-Wert von 1 bis 2 ein und extrahiert kontinuierlich, beispielsweise mit tert.-Butylether oder Butylacetat.
Aus der organischen Phase gewinnt man nach Trocknen, Filtrieren und Entfernen des Lösungsmittels 38,3 g zähen, öligen Rückstandes, der langsam kristallisiert.

### b) Herstellung von 2,3,6-Trifluorphenol (Vorprodukt)

Man legt den gemäß Beispiel 7a erhaltenen Rückstand (38,3 g) mit 150 g N-Methylpyrrolidon und 0,4 g Kupfer(I)oxid unter Rühren vor und erhitzt 3,5 Stunden auf 150 bis 160°C. Dabei decarboxyliert die eingesetzte 3-Hydroxy-2,4,5-trifluorbenzoesäure unter Bildung von 2,3,6-Trifluorphenol. Anschließend arbeitet man, wie in Beispiel 1b beschrieben, extraktiv mit Dichlormethan auf und erhält nach Entfernen des Lösungsmittels 14,7 g eines rohen, orangefarbenen Öl, das, bestimmt durch GC-Analyse, 94 % 2,3,6-Trifluorphenol (0,093 Mol entsprechend 57 % Ausbeute, bezogen auf die als Zwischenstufe nach Beispiel 7a hergestellte 3-Hydroxy-2,4,5-trifluorbenzoesäure) enthält.

## Patentansprüche

1. Verbindungen der Formel (1) 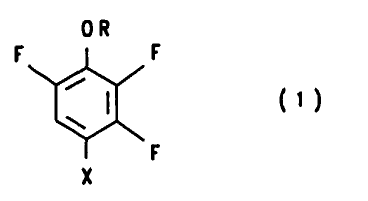 worin R für H, eine geradkettigen oder verzweigten Alkylrest mit 1 bis 6 Kohlenstoffatomen, eine fluorierten geradkettigen oder verzweigten Alkylrest mit 1 bis 6 Kohlenstoffatomen, einen Benzylrest oder einen durch eine Alkylgruppe oder Alkoxygruppe mit jeweils 1 bis 4 Kohlenstoffatomen oder Halogen substituierten Benzylrest steht, X für H, Cl, Br oder J steht, und X verschieden von R ist.

2. Verbindungen nach Anspruch 1, dadurch gekennzeichnet, daß R für H, einen geradkettigen oder verzweigten Alkylrest mit 1 bis 6 Kohlenstoffatomen oder einen Benzylrest steht.

3. Verbindungen nach Anspruch 1 oder 2, dadurch gekennzeichnet, daß R für einen geradkettigen oder verzweigten Alkylrest mit 1 bis 4 Kohlenstoffatomen oder einen Benzylrest steht.

4. Verbindungen nach einem oder mehreren der Ansprüche 1 bis 3, dadurch gekennzeichnet, daß X für H, Cl oder Br steht.

5. Verfahren zur Herstellung der Verbindungen der Formel (1) nach einem oder mehreren der Ansprüche 1 bis 4, worin R und X die vorstehend genannte Bedeutung besitzen, dadurch gekennzeichnet, daß man eine Verbindung der allgemeinen Formel (2) 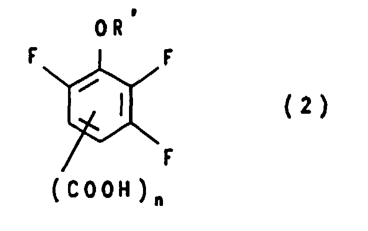 worin R' diesselbe Bedeutung wie R hat, oder, abweichend hiervon, für H steht und n = 1 oder 2 ist, in einem basischen Lösungsmittel oder Lösungsmittelgemisch in Anwesenheit eines Decarboxylierungskatalysators bei 120 bis 220°C decarboxyliert, das Reaktionsgemisch gegebenenfalls ansäuert, das decarboxylierte Produkt abtrennt und gegebenenfalls in das decarboxylierte Produkt den Rest X = Cl, Br oder J durch Halogenierung und gegebenenfalls den Rest R, sofern dieser nicht gleich H ist, durch Veretherung einführt.

6. Verfahren nach Anspruch 5, dadurch gekennzeichnet, daß man eine Verbindung der Formel (2), worin R' diesselbe Bedeutung wie R hat, einsetzt.

7. Verfahren nach Anspruch 5 oder 6, dadurch gekennzeichnet, daß man eine Verbindung der Formel (2), worin n = 1 ist, einsetzt.

8. Verfahren nach einem oder mehreren der Ansprüche 5 bis 7, dadurch gekennzeichnet, daß man ein basisches dipolar aprotisches Lösungsmittel, ein Alkylamin mit 6 bis 30 Kohlenstoffatomen ein Dialkylamin mit 6 bis 30 Kohlenstoffatomen je Alkylrest, ein Trialkylamin mit 4 bis 30 Kohlenstoffatomen je Alkylrest, eine N-enthaltende heterocyclische Verbindung oder ein Gemisch dieser vorstehend genannten Stoffe als basisches Lösungsmittel oder Lösungsmittelgemisch einsetzt.

9. Verfahren nach einem oder mehreren der Ansprüche 5 bis 8, dadurch gekennzeichnet, daß man als basisches dipolar aprotisches Lösungsmittel N-Methylpyrrolidon, Dimethylacetamid und 1,3-Dimethylimidazolidin-2-on oder Mischungen dieser Stoffe einsetzt.

10. Verfahren nach einem oder mehreren der Ansprüche 5 bis 9, dadurch gekennzeichnet, daß man ein Alkylamin mit 8 bis 20, insbesondere 8 bis 14 Kohlenstoffatomen, ein Dialkylamin mit 8 bis 20, insbesondere 8 bis 16 Kohlenstoffatomen je Alkylrest, ein Trialkylamin mit 6 bis 20, insbesondere 6 bis 14 Kohlenstoffatomen je Alkylrest oder Mischungen dieser Amine einsetzt.

11. Verfahren nach mindestens einem oder mehreren der Ansprüche 5 bis 10, dadurch gekennzeichnet, daß man als N-enthaltende heterocyclische Verbindung Pyridin, ein alkyliertes Pyridin, Chinolin, ein alkyliertes Chinolin, Isochinolin, ein alkyliertes Isochinolin oder ein Gemisch dieser Stoffe einsetzt.

12. Verfahren nach einem oder mehrere der Ansprüche 5 bis 11, dadurch gekennzeichnet, daß man ein basisches Lösungsmittel und Wasser als Lösungsmittelgemisch einsetzt.

13. Verfahren nach einem oder mehreren der Ansprüche 5 bis 12, dadurch gekennzeichnet, daß man als Decarboxylierungskatalysator Kupfer, eine Kupfer(I)verbindung, eine Kupfer(II)verbindung, beispielsweise Kupfer(I)oxid, Kupfer(II)oxid, Kupfer(I)sulfat, Kupfer(II)sulfat, Kupfer(I)chlorid, Kupfer(II)chlorid, Kupfer(I)fluorid, Kupfer(II)fluorid, Kupfercarbonat, Kupfer(I)hydroxid oder Kupfer(II)hydroxid einsetzt.

14. Verfahren nach einem oder mehreren der Ansprüche 5 bis 13, dadurch gekennzeichnet, daß man 0,1 bis 10, insbesondere 0,5 bis 3 Gew.-% Decarboxylierungskatalysator, bezogen auf die Verbindung der Formel (2), einsetzt.

15. Verfahren nach einem oder mehreren der Ansprüche 5 bis 14, dadurch gekennzeichnet, daß man die Decarboxylierung bei einem pH-Wert von 5 bis 8, durchführt.

16. Verfahren nach einem oder mehreren der Ansprüche 5 bis 15, dadurch gekennzeichnet, daß man die Decarboxylierung bei einer Temperatur von 130 bis 190, insbesondere 140 bis 170°C, durchführt.

17. Verfahren nach einem oder mehreren der Ansprüche 5 bis 16, dadurch gekennzeichnet, daß man das decarboxylierte Produkt mittels Extraktion oder Wasserdampfdestillation abtrennt.

18. Verfahren nach einem oder mehreren der Ansprüche 5 bis 17, dadurch gekennzeichnet, daß man das decarboxylierte Produkt in Anwesenheit oder Abwesenheit eines Lösungsmittels mit elementaren Halogen oder einem Hypohalogenit in Anwesenheit oder Abwesenheit eines Halogenierungskatalysator bei 70 bis 80°C umsetzt.

19. Verfahren nach einem oder mehreren der Ansprüche 5 bis 18, dadurch gekennzeichnet, daß man das decarboxylierte Produkt in Form des Phenolats mit einer Halogenverbindung R-Hal oder einem Sulfat der Formel (RO)₂SO₂ umsetzt.
